# EUROPEAN PATENT APPLICATION

(11) **EP 2 348 111 A1**
(43) Date of publication of application: **27.07.2011**
(21) Application number: 09821586.6
(22) Date of filing: 19.10.2009
(51) Int. Cl.: C12N 15/113, C12Q 1/68

(54) **PROBES FOR DETECTING MUTATIONS OF kRas GENE, LIQUICHIP AND DETECTION METHODS THEREOF**

(30) Priority: 21.10.2008 CN 200810218430
(71) Applicant: Guangzhou Surexam Bio-Tech Co., Ltd., Guangdong 510663 (CN)
(72) Inventor: XU, Jiasen, Guangzhou Guangdong 510663 (CN); WU, Shiyang, Guangzhou Guangdong 510663 (CN); REN, Lifen, Guangzhou Guangdong 510663 (CN); HE, Jiaying, Guangzhou Guangdong 510663 (CN); YANG, Huiyi, Guangzhou Guangdong 510663 (CN); LIN, Yiqun, Guangzhou Guangdong 510663 (CN)
(74) Representative: Bottero, Carlo
(86) International application number: PCT/CN2009/074508
(87) International publication number: WO 2010/045865

(57) **Abstract**

Nucleic acid probes for detecting kRas gene mutations, liquid chips and detection methods thereof are provided. The liquid chips for detecting kRas gene mutations comprise: microspheres coupled with wild-type and mutant-type probes, each of which is amino-substituted at 5'-terminal, specific for kRas codons 12, 13 and/or 61, and primers for amplifying the target sequence biotin-labeled at the terminal which are enriched with mutant alleles of kRas codons 12, 13 and/or 61 to be detected. The detection methods are rapid and accurate, and the processes of the methods are easy to perform. The liquid chip can be used to detect mutations of kRas as assistance to early diagnosis of pancreatic cancer, and can be used to prognose efficiency of the molecular targeted therapy to choose right medicine accurately clinically and avoiding economic loss and time loss caused by unnecessary treatment.

## Description

### FIELD OF THE INVENTION

The present invention relates to the field of biotechnology, and in particular to probes for detecting kRas gene mutations, liquid chips and detection methods thereof.

### BACKGROUND OF THE INVENTION

### 1. kRas gene mutation and early diagnosis of pancreatic cancer

Ras genes are common oncogenes in human tumors. The Ras gene family consists of kRas, hRas and nRas, and each member of the gene family shares 85% of homology with respect to each other. The Ras gene-encoded protein P21 with GTPase activity is located at the inner membrane, and participates in the regulatory system of the signal transduction of cell proliferation. Its active state is the GTP binding state, and its inactive state is the GDP binding state. The main parts of the P21 protein which transform into active oncogene are the mutations of the codons 12, 13 and 61. The activation of kRas gene in the occurring of pancreatic cancer has been confirmed in studies, and the most familiar is the mutation of the kRas codon 12. The mutant kRas P21 protein exhibited low GTPase activity that can not dissociate GTP quickly, and therefore ras signal transduction protein "locks" in the active GTP binding state. The ras signal protein in active state results in uncontrollable proliferation and canceration of cells through cell signal transduction pathway.

The mutation rate of kRas gene varies in different tumor tissues. The mutation rate of kRas gene is 43% in the carcinoma of colon, 30%in lung cancer, 29% in thyroid cancer, 10% or even lower in cancers of bladder, liver, kidney and uterus.

In the pancreatic cancer, the mutation rate of kRas gene is 75 %-95 %, the mutation almost always happens in the codon 12 in the form of CGT, GTT and GAT, and there is no K-ras gene mutations in normal pancreatic tissue, acute and chronic pancreatitis, insulinoma, benign pancreatic cysts and other diseases of pancreas. Meanwhile, the kRas gene mutation is a pattern of the pancreatic cancer in the early stage, and the mutation rate during the tumor formation varies as follows: 26% in the proliferative stage, 46% in the proliferative stage of nipple, 76% in the stage of carcinoma in situ, 80% in the stage of adenocarcinoma, and 43% in the stage of lymph node metastasis. Therefore, detecting the mutation of kRas codon 12 would be helpful to assist in diagnosing pancreatic cancer, and determining benign and malignant nature of pancreatic masses. It is an important auxiliary means for diagnosing pancreatic cancer by detecting the mutation of K-ras codon 12 in the pancreatic juice, gall, duodenal juice, dejecta and peripheral blood.

### 2. kRas gene mutation and Primary drug resistance of non-small cell lung cancer (NSCLC) against the target therapy drugs such as Gefitinib and Erlotinib.

The kRas gene is a crucial downstream regulatory molecule in the signal transduction pathway of the epidermal growth factor receptor (EGFR The EGFR is a multifunctional glycoprotein widely existing on cell membranes of various human tissues, and has tyrosine kinase activity, and it is one of the four members of ErbB family, thus it is also known as HER1 or ErbB1. Activated EGFR induced by ligands start intracellular signal cascades composed of cytoplasmic enzymes and adapter proteins. The subsequent signal activates transcription factors and finally directs cellular responses, including migration, adhesion, proliferation, differentiation and apoptosis. Research shows that many solid tumors have abnormal or high expression of EGFR, which provides theoretical basis and experimental evidence for signal transduction interference therapy in connection with EGFR signal transduction pathway and for tumor therapy by targeting EGFR. Accordingly, molecular targeted therapy in which the EGFR is the therapeutic target has been an international focus in the field of oncology. The EGFR tyrosine kinase inhibitors Genfitinib (Irressa) and Erlotinib (Tarceva) have been approved by U.S. Food and Drug Administration to be used in the therapy of advanced non-small cell lung cancer (NSCLC). In February 2005, the Genfitinib was also approved to be used in clinical application in China. These drugs have also been used in clinical test for treating other tumors such as breast cancer, colon cancer, and gastric cancer.

The Genfitinib and Erlotinib are oral small molecular EGFR antagonists, and can compete with ATP to bind to ATP binding pockets in EGFR tyrosine kinase zone, so as to inhibit the EGFR activity. They have been used in the clinical treatment of patients whose non-small cell lung cancer is advanced and not suitable for traditional chemotherapy, and they are the most effective drugs for some of the NSCLC patients. However, the reaction of patients to this kind of molecular targeted drugs is related to individual genetic background. In June 2004, researchers Lynch, Paez, etc., in Harvard Medical School reported that the gene mutation in EGFR tyrosine kinase encoding region of lung cancer cells is a necessary precondition to enable the targeted therapy drugs to be effective. Research indicates that the effective rate of Genfitinib reaches up to 80% in therapy of the tumor of the type of mutations in EGFR tyrosine kinase encoding region, while it is ineffective with respect to the wild type tumor without mutations. This research result receives high attention from scholars in the world, and has been approved by institutes in USA, Japan, Korea and China in less than one year (referring to Pao W, et al, 2004; Han SW, et al, 2005; Mitsudomi T, et al, 2005; Huang SF, et al, 2004; Mu XL, et al, 2005).

The deletion mutation of exon 19 in EGFR and the point mutation of exon 21 enable the patients to be sensitive to the targeted therapy drugs such as Gefitinib and Erlotinib, while other mutations enable the patients to be resistant to these drugs, which is also called as drug resistance. The drug resistance includes primary drug resistance and secondary drug resistance (acquired drug resistance). The T790 point mutation of exon 20 in EGFR is related to the secondary drug resistance of the targeted therapy drugs. The mutation of kRas gene encoding GTPase at the downstream of the EGFR signal transduction pathway is caused by the primary drug resistance of Gefitinib and Erlotinib. About 15-30% of NSCLC patients has kRas gene point mutation, and is related to the poor prognosis of the targeted therapy of patients. These mutations are mostly located at the codons 12 and 13 of the second exon. Research indicates that the NSCLC with kRas gene mutation has poor sensitivity to the tyrosine kinase inhibitors Erlotinib and Gefitinib. This finding is firstly reported by William Pao, et al. The researchers selected 60 adenocarcinoma patients who are sensitive and not sensitive to Gefitinib and Erlotinib, and detecting their EGFR gene mutation and kRas gene mutation. In all the patients who are not sensitive to the Gefitinib and Erlotinib, 24% (9/38) patients have mutation in kRas gene, while in all the patients who are sensitive to the Gefitinib and Erlotinib, none of them (0/21) has mutation in kRas gene.

In 2006, Baoli Qin, et al, studied the relationship between the key molecules of the EGFR signal transduction pathway and the drug resistance of the targeted therapy of Gefitinib. It was found that the activation of the Src and Ras genes can increase the drug resistance of tumor cells to Gefitinib by 30 to 200 times. In addition, Sae-Won Han, et al, in Korea (Clinical Cancer Res 2006; 12(8)) selected 69 NSCLC patients who received therapy of Gefitinib, wand detected the EGFR gene copy number, the K-ras gene mutation, HER2, the mutation of EGFR exon 18-21, and the condition of the Akt phosphorylation to study the factors relative to the sensitivity of Gefitinib. The analysis results show that the patients with kRas gene mutation are not sensitive to Gefitinib. Among the EGFR wild-type patients, all the patients carrying kRas gene mutation or p-Akt over-expression has relatively bad therapy effect, and it is easy to have a relapse (P=0.017). Generally speaking, the kRas gene mutation and the EGFR gene mutation are repulsive with respect to each other, the EGFR gene mutation is mainly found in a person who does not smoke, while the kRas gene mutation is mainly found in the cancer relative to smoking.

### 3. kRas gene mutation and drug resistance of colorectal cancer against Cetuximab

The kRas gene mutation is not only relative to the primary drug resistance of the non-small cell lung cancer to the EGFR tyrosine kinase inhibitors such as Gefitinib and Erlotinib. Recently, it is also found that this kRas gene mutation is also closely related to the drug resistance of colorectal cancer to Cetuximab.

The EGFR targeted drug Cetuximab is effective to the colorectal cancer that is difficult to cure, and it is a benefit to overcome the drug resistance to Irinotecan when it is used in the chemotherapy scheme which mainly uses Irinotecan. However, only some of the patients benefit from Cetuximab therapy, and hence the determination of curative effect factor is very important. Lievre, et al, in University Paris Descartes in France reported that when Cetuximab was used in the therapy of patients with metastatic colorectal cancer, the kRas gene mutation in tumor can prognose the effectiveness in therapy and the survival rate of patients (Cancer Res 2006; 66: (8)).

The researchers found that the kRas gene mutation in tumor cells was related to the drug resistance of Cetuximab and the relatively short overall survival (OS). Among 30 patients with metastatic colorectal cancer who receive therapy with Cetuximab, the mutation condition of kRas, BRAF and PIK3CA genes was detected through sequencing method. Among the 30 patients, Cetuximab was effective to 11 patients, while there was no therapeutic response to 19 patients. In the 19 patients, 68.4% patients had kRas gene mutation, and no kRas gene mutation existed in the 11 patients effective to Cetuximab. Meanwhile, the overall survival of the patients without kRas gene mutation was significantly different from that of the patients with kRas gene mutation (16.3 months and 6.9 months respectively, P=0.016).

On that basis, the researchers included retrospective analysis on 89 patients with metastatic colorectal cancer and drug resistance against Irinotecan [J Clin Oncol 2008, 26 (3):374]. The patients received therapy of Irinotecan plus Cetuximab (88%), Cetuximab plus fluorouracil and folic acid (10%), and simply Cetuximab (2%) respectively.

Immunohistochemical results indicated that all the patients had high-expression of EGFR. Generally, Cetuximab is effective to 26 patients. Getuximab is not effective to the 24 patients with kRas mutations, and the patients who are effective to the therapy are all the patients without mutations (26/65, P<0.001). The patients without mutations have the progress free survival (PFS) and the overall survival (OS) which are longer than the patients with mutations (PFS:31.4 weeks vs 10.1 weeks, p=0.0001; OS: 14.3 months vs 10.1 months, p=0.026).

This research result is identical to that of the other above-mentioned researches, and teaches that before the therapy to the patients with refractory colorectal cancer by using Cetuximab, the kRas mutation should be fully considered, which may be good for reducing the therapy cost, and avoid the unnecessary use of the expensive reagents and the influence of toxicity of the unnecessary treatment.

### 4. The development of kRas gene mutation detecting reagent

At present, most of the detecting products of the kRas gene mutation use the method of real-time PCR, and the lowest detection limit is 1-2% of the mutant-type in the wild-type, and also this method has a relatively high false positive rate. The product for detecting kRas gene mutation by using liquid chip technology is blank in the world.

In addition, for the helerogeneity of the tumor, there may be only a little amount of mutant genes and a large amount of wild type genes in the free nucleic acid. The large amount of wild type genes will disturb the detection of mutant genes. Therefore, it is a key problem firstly needed to be solved that how to remove the disturbing of the wild type sequence to the detection of the kRas gene mutation.

### SUMMARY OF THE INVENTION

It is an object of the present invention to provide probes for detecting kRas gene mutations.

The above object is achieved by the following technical solutions:
Probes for detecting kRas gene mutations, comprise:
   specific for kRas codon 12: a wild-type probe of 5'GAGCTGGTGGCGTAGGCAAG3', and a wild-type probe of 5' GACCTGGTGGCGTAGGCAAG3'introducing restriction site BstOI, and a mutant-type probe of one or more nucleotide sequences introducing restriction site BstOI selected from 5' GACCTCGTGGCGTAGGCAAG3', 5'GACCTAGTGGCGTAGGCAAG3', 5'GACCTTGTGGCGTAGGCAAG3', 5'GACCTGATGGCGTAGGCAAG3', 5'GACCTGCTGGCGTAGGCAAG3', and 5'GACCTGTTGGCGTAGGCAAG3'; and/ or
   specific for kRas codon 13: a wild-type probe of 5' AGCTGGTGGCGTAGGCAAGA3', and a wild-type probe of 5' AGCTGGTGGCGCCGGCAAGA3' introducing restriction site NarI, and a mutant-type probe of one or both nucleotide sequences introducing restriction site NarI selected from 5' AGCTGGTGACGCCGGCAAGA3' and 5' AGCTGGTTGCGCCGGCAAGA3'; and/ or;
   specific for kRas codon 61: a wild-type probe of 5'AGCAGGTCAAGAGGAGTACAGT3', a wild-type probe of 5' AGCTGATCAAGAGGAGTACAGT3' introducing restriction site BclI and 5' AGCAGGTCAAGATCTGTACAGT3' introducing restriction site Bg1II, and a mutant-type probe of one or more nucleotide sequences selected from 5' AGCTGATAAAGAGGAGTACAGT3'introducing restriction site BclI, 5' AGCTGATCTAGAGGAGTACAGT3' introducing restriction site BelI, and 5' AGCAGGTCATGATCTGTACAGT3' introducing restriction site BglII.

It is a further object of the present invention to provide a liquid chip for detecting kRas gene mutation. This liquid chip can be used for detecting the corresponding mutations of kRas codons 12, 13 and/or 61 (codons 12, 13 are located at exon 2, and codon 61 is located at exon 3). This liquid chip can not only be used in early diagnosis of pancreatic cancer, but also in prognosing the curative effect of EGFR tyrosine kinases inhibitors to treat NSCLC such as Gefinitnib and Eelotinib, and the curative effect of Panitumumab or Cetuximab to the colorectal cancer.

The above object is achieved by the following technical solutions:
A liquid chip for detecting kRas gene mutations comprises:
   A. microspheres coupled with probes:
      microspheres respectively coupled with amino-substituted wild-type probes specific for kRas codon 12, wherein the wild-type probes are SEQ ID NO. 1 and SEQ ID NO. 2; and microspheres respectively coupled with amino-substituted mutant-type probes specific for kRas codon 12, wherein the mutant-type probes are one or more nucleotide sequences selected from SEQ ID NO. 3- SEQ ID NO. 8; and/or
      microspheres respectively coupled with amino-substituted wild-type probes specific for kRas codon 13, wherein the wild-type probes are nucleotide sequences SEQ ID NO.9 and SEQ ID NO.10; and microspheres respectively coupled with amino-substituted mutant-type probes specific for kRas codon 13, wherein the mutant-type probes are one or both nucleotide sequences selected from SEQ ID NO.11 and SEQ ID NO.12; and/or
      microspheres respectively coupled with amino-substituted wild-type probes specific for kRas codon 61, wherein the wild-type probes are nucleotide sequences SEQ ID NO.13 SEQ ID NO.14 and SEQ ID NO.15; and microspheres respectively coupled with amino-substituted mutant-type probes specific for kRas codon 61, wherein the mutant-type probes are one or more nucleotide sequences selected from SEQ ID NO.16-17 introducing restriction site BclI, and SEQ ID NO.18 introducing restriction site BglII;
      wherein a spacer is connected between the nucleotide sequence of each kind of the above-mentioned probes and the amino group, and each kind of the microspheres has different color code; and
   B. primers: primers for amplifying the target sequence enriched for mutant alleles of kRas codons 12, 13 and/or 61 to be detected, the target sequence is biotin-labeled at the terminal.

The spacer is the sequence used for separating the specific probe from the microsphere surface or placing the specific probe into the hydrophilic environment. By providing an appropriate length of spacer sequence between the probe sequence and the amino group, the steric hindrance is reduced, and the efficiency of hybridization and the specificity of hybridization are improved. The familiar spacer sequences include poly (dT), oligomer polyethylene glycol and (CH2)n spacer (n≥3), such as ( CH2 ) 12, ( CH2 ) 18; etc. In addition, if the disturbing of poly ( dA ) exists, it can also use the poly ( TTG ) to be the spacer. Preferably, the spacer is an oligonucleotide consisting of 5 to 40 deoxythymidylates , and more preferably 10 deoxythymidylates .

Preferably, the primers for amplifying the target sequences enriched for mutant alleles of codons 12, 13 and/or 61 include:
primers for amplifying the target sequences enriched for mutant alleles of kRas codons 12, wherein the primers are SEQ ID NO. 19 - SEQ ID NO. 21, and at least one of the primers is biotin-labeled at the terminal; and/or
primers for amplifying the target sequences enriched for mutant alleles of kRas codons 13, wherein the primers are SEQ ID NO. 22 - SEQ ID NO. 25, and at least one of the primers is biotin-labeled at the terminal; and/or
primers for amplifying the target sequences enriched for mutant alleles of kRas codons 61, wherein the primers are SEQ ID NO. 26 - SEQ ID NO. 32, and at least one of the primers is biotin-labeled at the terminal.

It is a further object of the present invention to provide a method for detecting the kRas gene mutations, which is rapid, accurate and easy to operate.

A method for detecting kRas gene mutations, using the liquid chip for detecting mutation of kRas gene, comprises the following steps:
(1) performing a first PCR amplification for wild-type and mutant-type DNA samples by using PCR primers with resctriction site;
(2) performing restriction digestion to products obtained from the first PCR amplification of the step (1);
(3) performing a second PCR amplification for the mutant-type kRas gene by using products obtained after the restriction digestion as template;
(4) hybridizing products obtained from the second PCR amplification to the corresponding probes coupled to microspheres of claim 2;
(5) performing reaction by adding streptavidin-phycoerythrin after the hybridization reaction of the step (4), and then performing signal detection.

The present invention has the following advantages:
(1) by using the liquid chip for detecting the kRas gene mutations, the alleles with a relatively high mutation frequency in kRas gene can be simultaneously detected, the reaction condition is uniform during detection, and the detection results are more specific, sensitive, and with more than 99% accuracy.
(2) the detection method of the present invention is simplified in operation, which avoids uncertainty factors during the complicated operation process, and hence the accuracy of detection is highly improved.
(3) the time required in the detection method of the present invention is much less than that of normal sequencing technique, which is especially adapted to the clinic use.
(4) the present invention uses the method of introducing restriction site and then enriching by restriction digestion so as to amplify the target mutant-type sequence and then to be used in detection, which prevents the large amount of wild-type sequences in the products from disturbing the detection results.
(5) the liquid chip and the detection method of the present invention can not only detect the kRas gene mutations in tumor tissue sample, but also can detect the kRas gene mutations in the body fluid of the tumor patients, by which the sampling is easy, the pain suffered by patients is reduced, and it can be dynamically monitored.

### BRIEF DESCRIPTION OF THE DRAWINGS

Fig. 1 is a schematic diagram showing the sequence information of mutant region of exon 2 and exon 3;
Fig. 2 is a schematic diagram showing 6 kinds of mutations of codon 12 enriched by restriction digestion of BstOI;
Fig. 3 is a schematic diagram showing 2 kinds of mutations of codon 13 enriched by restriction digestion of NarI;
Fig. 4 is a schematic diagram showing 3 kinds of mutations of codon 61 enriched by restriction digestion;
Fig. 5 is a schematic diagram of the analysis on the restriction digestion results in the Embodiment 2;
Fig. 6 is a schematic diagram of the analysis on the results of sensitivity test of kRas codon 12;
Fig. 7 is a schematic diagram of the analysis on the results of the test in which the mutations are detected from the wild-type plasmid of kRas codon 13.

### DETAILED DESCRIPTION OF THE PREFERRED EMBODIMENT

In the EGFR program developed in the past, a large amount of wild-type genes are removed by endonuclease, and then the mutant-type genes are amplified by PCR. However, three mutant alleles of kRas gene (the codons 12, 13 of the exon 2, and the codon 61 of the exon 3) have no corresponding restriction site to remove the wild-type genes. In this test, the PCR technique is used to introduce the restriction site, and then the wild-type genes are removed by restriction digestion such that the mutant sequences are enriched.

### 1) 1^{st} PCR introduces the restriction site

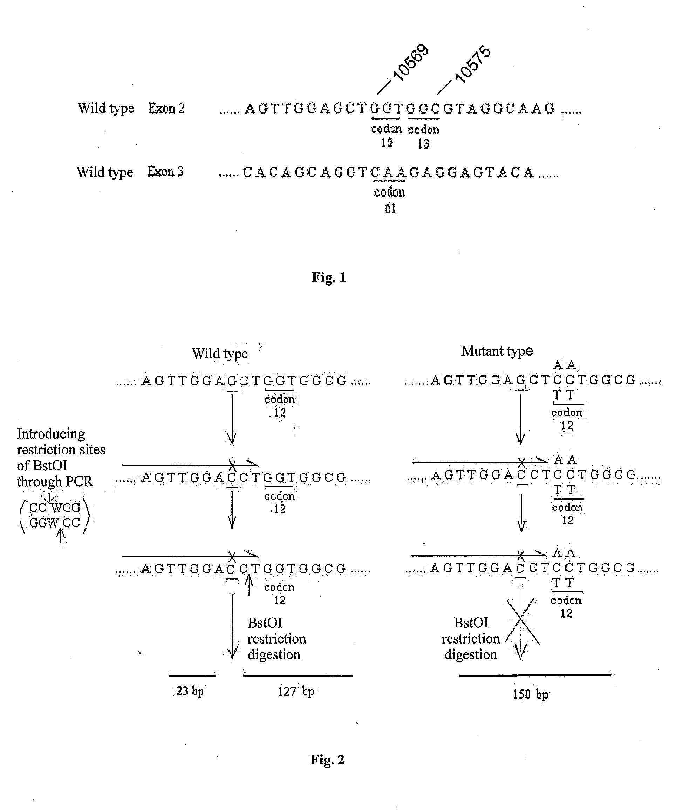

The main mutant alleles of kRas genes transforming into active oncogenes are the codons 12, 13 and 61. The codons 12, 13 are located at exon 2 (Fig. 1), and the codon 61 is located at exon 3 (referring to Fig. 1).

Generally, there are 6 kinds of mutations in kRas codon 12 (the 10569bp to 10571bp of kRas gene): GGT >GAT , GGT->GCT , GGT->GTT , GGT->AGT , GGT->CGT , GGT- >TGT. The introducing of the restriction site at the codon 12 is performed by transforming "G" of 10566bp into "C", so that the restriction site of BstOI(BstNI) is introduced (referring to Fig. 2). To reduce the risk, another approach can be used, that is, "G" of 10566bp is transformed into "A", and introducing the restriction site of BselI(BsrSI) to remove the wild type. A pair of primers is designed, and about 150bp fragment is amplified to enrich the fragment containing the codon 12, and the wild type is removed by restriction digestion, and 6 kinds of mutant types are enriched.

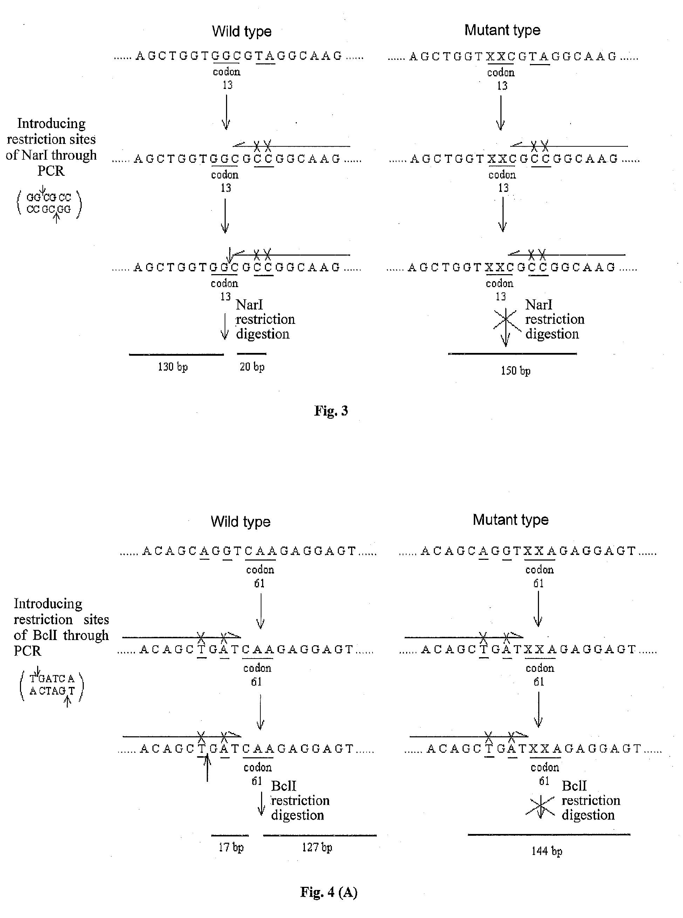

There are 2 kinds of mutation of kRas codon 13 (the 10572bp to 10574bp of kRas gene) in general: GGC->GAC , GGC->TGC. The introducing of the restriction site at the codon 13 is performed by transforming "TA" of 10576-10577bp into "CC", so that the restriction site of NarI is introduced to remove the wild type. A pair of primers is designed, and about 150bp fragment is amplified to enrich the fragment containing the codon 13, and the wild type is removed by restriction digestion ofNarI, and 2 kinds of mutant types are enriched (referring to Fig. 3).

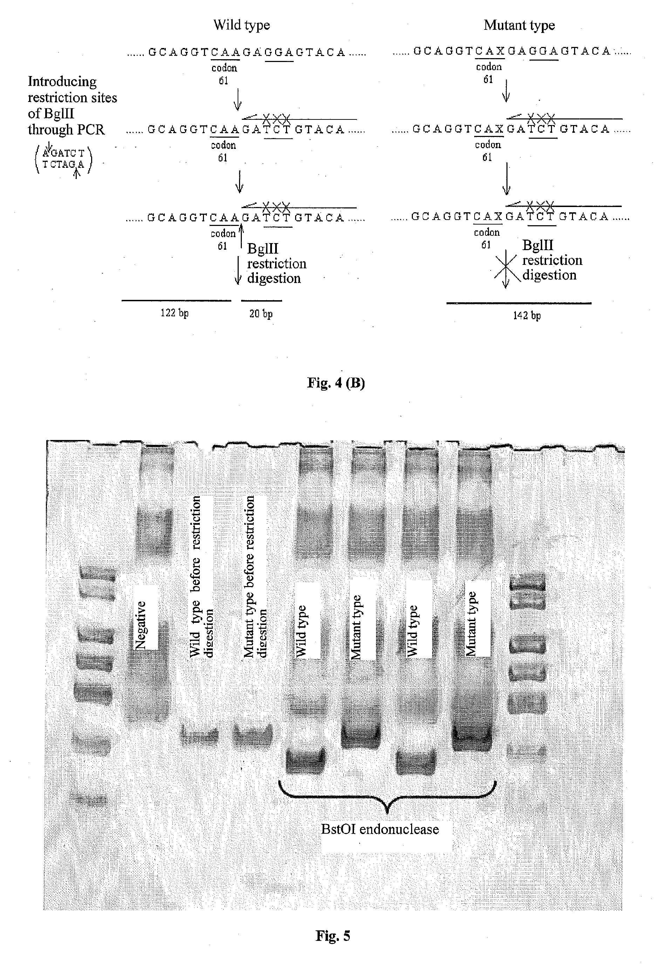

Generally, there are 3 kinds of mutations in kRas codon 61 (the 28578bp to 28580bp of kRas gene): CAA->AAA , CAA->CTA, CAA->CAT. Because the codon 61 can not be enriched for three kinds of mutant type by introducing single restriction site, and should introduce two restriction sites BclI("A"of 28574bp transform into"T","G"of 28576bp transform into"A") and BglII("GGA"of 28583-28585bp transform into"TCT") to remove the wild type. Two pairs of primers are designed to respectively amplify 144bp fragment from 28558bp to 28701 bp and 142bp fragment from 28459bp to 28600bp so as to enrich the fragments containing the codon 61, and two mutants CAA->AAA, CAA->CTA can be enriched by restriction digestion of BelI, and the mutant CAA->CAT can be enriched by restriction digestion of Bg1II (referring to Fig. 4).

The main materials required for the assay

### 1) plasmids containing the wild-type kRas gene(plasmid: exon 2 and exon 3) and 11 kinds of mutant alleles (table 1)

**Table 1 the plasmids containing the mutant alleles used in this experiment**

| exon | codon | mutant plasmid | mutant alleles |
|---|---|---|---|
| Exon 2 | Codon 12 | kRas-G12D | GGT->GAT |
| | | kRas-G12A | GGT->GCT |
| | | kRas-G 12V | GGT->GTT |
| | | kRas-G12S | GGT->AGT |
| | | kRas-G 12R | GGT->CGT |
| | | kRas-G12C | GGT->TGT |
| | Codon 13 | kRas-G13D | GGC->GAC |
| | | kRas-G13C | GGC->TGC |
| Exon 3 | Codon 61 | kRas N61K | CAA->AAA |
| | | kRas-N61L | CAA->CTA |
| | | kRas-N61H | CAA->CAT |

Solution Formulation : coupling buffer (pH4.5) : 0,1mol/L MES ( Sigma M-2933)
Washing buffer : 0.21ml/L Tween-20 (Sigma P-9416), 1g/L SDS ( Sigma L-4390 )
TE buffer pH8.0)(Storage solution):10mmol/L Tris(Sigma 337501) ,1mmol/LEDTA(Sigma E-5134 ) 2×Tm hybridization buffer

| Reagent | source | final concentration | Serving size per250ml |
|---|---|---|---|
| 1MTris-HCl, pH8.0 | SigmaT3038 | 0.2M | 50ml |
| 5M NaCl | Sigma S5150 | 0.4M | 20ml |
| Triton X-100 | Sigma T8787 | 0.16% | 0.4ml |

| | | | |
|---|---|---|---|
| After filtration, stored at 4°C. | | | |

### Embodiment 1

### Preparation of liquid chip for detecting kRas gene mutations

### I. The probes design and microsphere coupling

Specific oligonucleotide probes were designed for detection of the wild-type and mutant-type of kRas codons 12, 13 and 61. The detailed steps for each microsphere coupling are as follows:
(1) resuspend the stock uncoupled microsphere (purchased from Luminex Corporation), by vortex (vortex vibration) for 30sec, and sonication for 1min;
(2) transfer 8ul of the stock microspheres, which contains total of 0.8×10⁵ to 1.2×10⁵ microspheres, to a 0.5ml microfuge tube;
(3) pellet the stock microspheres by microcentrifugation at 15,000 rpm for 10min, and remove the supernatant carefully;
(4) add 10ul of the coupling solution (pH4.5), and mix by vortex for 30sec, and sonication for 1min;
(5) add 2ul of 2pmol/ul probe working solution;
(6) add 2.5ul of 5mg/ml EDC (1-(3-Dimethylaminopropyl)-3-ethylcarbodiinide hydrochloride working solution, and incubate at 25 °C in the dark for 30min; repeat this step once;
(7) add 0.2ml of washing buffer, perform vortex for 30sec and then pellet the coupled microspheres by microcentrifugation at 12,000g for 5min, and remove the supernatant carefully; repeat this step once;
(8) add 500ul of TE buffer, perform vortex for 30sec and sonication for 30sec;
(9) pellet the coupled microspheres by microcentrifugation at 12,000g for 5min, and remove the supernatant carefully;
(10) add 20ul of TE buffer, and stored under 2-8 °C.

The prepared liquid chips for detecting kRas gene mutations include: A. microspheres coupled with probes, the nucleotide sequence for each probe with microspheres coupled are as follows:

| **Microsphe res No.** | **Probes Names (mutation site)** | **Probe nucleotide sequences** | **SEQ NO** |
|---|---|---|---|
| 12 | 60C12W1 | 5'NH2-TTTTTTTTTTGAGCTGGTGGCGTAGGCAAG3' | **1** |
| 20 | 60C12WE1 ( GCT->CCT) | 5'NH2-TTTTTTTTTTGACCTGGTGGCGTAGGCAAG3' | **2** |
| 28 | 60C12MEE1 ( GGT->CGT ) | 5'NH2-TTTTTTTTTTGACCTCGTGGCGTAGGCAAG3' | 3 |
| 16 | 60C12ME2(GGT-> AGT) | 5'NH2-TTTTTTTTTTGACCTAGTGGCGTAGGCAAG3' | **4** |
| 18 | 60C12ME3(GGT-> TGT) | 5'NH2-TTTTTTTTTTGACCTTGTGGCGTAGGCAAG3' | **5** |
| 21 | 60C12ME4(GGT-> GAT) | 5'NH2-TTTTTTTTTTGACCTGATGGCGTAGGCAAG3' | **6** |
| 45 | 60C12ME5(GGT-> GCT) | 5'NH2-TTTTTTTTTTGACCTGCTGGCGTAGGCAAG3' | 7 |
| 63 | 60C12ME6(GGT-> GTT) | 5'NH2-TTTTTTTTTTGACCTGTTGGCGTAGGCAAG3' | 8 |
| 38 | 60C13W | 5'NH2-TTTTTTTTTTAGCTGGTGGCGTAGGCAAGA3' | **9** |
| 53 | 60C13WN(GTA-> GCC) | 5'NH2-TTTTTTTTTTAGCTGGTGGCGCCGGCAAGA3' | **10** |
| 72 | 60C13MN1(GGC- | 5'NH2-TTTTTTTTTTAGCTGGTGACGCCGGCAAGA3' | **11** |
| | >GAC) | | |
| 66 | 60C13MN2(GGC- >TGC) | 5'NH2-TTTTTTTTTTAGCTGGTTGCGCCGGCAAGA3' | **12** |
| 22 | C61W1 | 5'NH2-TTTTTTTTTTAGCAGGTCAAGAGGAGTACAGT3' | **13** |
| 25 | C61WBc1(AGG-> TGA) | 5'NH2-TTTTTTTTTTAGCTGATCAAGAGGAGTACAGT3' | **14** |
| 32 | C61MBc1(CAA-> AAA) | 5'NH2-TTTTTTTTTTAGCTGATAAAGAGGAGTACAGT3' | **16** |
| 36 | C61MBc2(CAA-> CTA) | 5'NH2-TTTTTTTTTTAGCTGATCTAGAGGAGTACAGT3' | **17** |
| 76 | C61WBg1(GGA-> TCT) | 5'NH2-TTTTTTTTTTAGCAGGTCAAGATCTGTACAGT3' | **15** |
| 77 | C61MBg1(CAA-> CAT) | 5'NH2-TTTTTTTTTTAGCAGGTCATGATCTGTACAGT3' | **18** |

B: Primers: the primers for amplifying the target sequences enriched for mutant alleles of the kRas codons 12, 13 and 61 to be tested, and the amplified target sequence can be biotin-labeled at the terminal, the nucleotide sequence of each primer is as follows:

| Primers Name | codon | nucleotide sequences | First Round or Second round | **SEQ.** |
|---|---|---|---|---|
| K12EF | Codon 12 | 5'ATATAAACTTGTGGTAGTTGGACCT3' | First Round and Second round | 19 |
| K12R1 | Codon 12 | 5' CTGTATCAAAGAATGGTCCTGC 3' | First Round | 20 |
| K12R-bio | Codon 12 | 5' biotin-ATGGTCCTGCACCAGTAATATG 3' | Second round | 21 |
| K13R | Codon 13 | 5' CGTCAAGGCACTCTTGCCGGCG 3' | First Round | 22 |
| K13R-bio | Codon 13 | 5'biotin-CGTCAAGGCACTCTTGCCGGCG3' | Second round | 23 |
| K13F1 | Codon 13 | 5' TGGAGTATTTGATAGTGTATTAACC 3' | First Round | 24 |
| K13F2 | Codon 13 | 5' TGATAGTGTATTAACCTTATGTGTG 3' | Second round | 25 |
| K61BcF | Codon 61 | 5' TTGGATATTCTCGACACAGCTGAT 3' | First Round and Second round | 26 |
| K61BcR1 | Codon 61 | 5' AATTTAAACCCACCTATAATGGTG 3' | First Round | 27 |
| K61BcR1-b | Codon 61 | 5' biotin-CCACCTATAATGGTGAATATCTTC 3' | Second round | 28 |
| K61BgR1 | Codon 61 | 5' GTCCCTCATTGCACTGTACAGATC 3' | First Round | 29 |
| K61BgR1-b | Codon 61 | 5'biotin-GTCCCTCATTGCACTGTACAGATC 3' | Second round | 30 |
| K61BgF1 | Codon 61 | 5' GTAAAAGGTGCACTGTAATAATCC 3' | First Round | 31 |
| K61BgF2 | Codon 61 | 5' TAATCCAGACTGTGTTTCTCCC 3' | Second round | 32 |

### Embodiment 2, Introducing restriction sites to kRas codon 12 by PCR, and the removal of the

### wild-type

Specific for the sequences of kRas codon 12, a pair of primers with restriction sites of BstOI is designed to amplify the wild-type and mutant-type, and the wild-type will be removed effectively by restriction digestion, but the mutant-type will not be removed, so as to achieve the purpose of mutant enrichment.

### 1. The PCR amplification

### PCR reaction system

| **Codon 12 reaction system** | **Per reaction (µl)** |
|---|---|
| Sterile ddH₂O | 28.8 |
| 5xColorless GoTaq Flexi Buffer | 10 |
| dNTP Mixture (each 2.5mM) | 2 |
| MgCl₂ (25mM) | 5 |
| Primer F : K12EF(10uM) | 1 |
| Primer R : K12R1(10uM) | 1 |
| GoTaq Hot Start-polymerase ( 5U/ul) | 0.2 |
| DNA Templates | 2 |
| total volume | 50 |

### PCR Reaction condition :

| **Steps** | **Temperature** | **Time** | **cycle numbers** |
|---|---|---|---|
| Initial denaturation | 94°C | 3 min | 1 |
| Denaturation | 94°C | 20 sec | 35 |
| Anneal | 58°C | 30 sec | |
| Extension | 72°C | 20 sec | |
| Final extension | 72°C | 10 min | 1 |
| Preservation | 4°C | ∞ | 1 |

### 2. BstOI restriction digestion of PCR Products:

### Reaction system is as follow:

| Sample system (Incubated at 60°C for 2hr) | Per reaction (µl) |
|---|---|
| 10×Buffer C | 5 |
| BstOI endonuclease (10U/ul) | 0.5 |
| BSA | 0.5 |
| PCR products | 10 |
| Sterile ddH₂O | 34 |
| Total volume | 50 |

Figure 5 shows the restriction digestion results. The results for the PAGE (polyacrylamide gel electrophoresis) showed that the wild-type codon 12 was completely digested, but the mutant-type was not digested. This result proves the feasibility for introduction of restriction site by PCR to remove the wild-type, and the enrichment of mutant-type.

### Embodiment 3, the sensitivity experiments of kRas codon 12

In order to determine the sensitivity of codon 12, take 1,3,9,27,81.243,729 copies of plasmid containing codon 12 mutant-type for detection, each experiment repeated 4 times.

### I. PCR amplification and restriction digestion

### 1. The first PCR amplification

PCR reaction system and the implementation are the same as Embodiment 2

### PCR reaction conditions:

| Steps | Temperature | Time | Cycle number |
|---|---|---|---|
| Initial denaturation | 94 °C | 3 min | 1 |
| Denaturation | 94 °C | 20 sec | 20 |
| Anneal | 58°C | 30 sec | |
| Extension | 72 °C | 20 sec | |
| Final Extension | 72 °C | 10 min | 1 |
| Preservation | 4°C | ∞ | 1 |

### 2. BstOI restriction digestion of PCR product :

### Reaction system is the same as embodiment 2.

### 3. The second PCR amplification

### PCR reaction system:

| Condon 12 reaction system | Per reaction (ul) |
|---|---|
| Sterile ddH2O | 28.8 |
| 5xColorless GoTaq Flexi Buffer | 10 |
| dNTP Mixture (each 2.5mM) | 2 |
| MgCl₂ (25mM) | 5 |
| Codon 12 primer F:K12EF(10uM) | 1 |
| Codon 12 primer R : K12R-bio(10uM) | 1 |
| GoTaq Hot Start polymerase ( 5U/ul ) | 0.2 |
| DNA Template | 2 |
| Total volume | 50 |

### PCR reaction conditions:

| Steps | Temperature | Time | cycle number |
|---|---|---|---|
| Initial denaturation | 94 °C | 3 min | 1 |
| Denaturation | 94 °C | 20 sec | 30 |
| Anneal | 60°C | 30 sec | |
| Extension | 72 °C | 20 sec | |
| Final extension | 72 °C | 10 min | 1 |
| Preservation | 4°C | ∞ | 1 |

### II. Luminex detection

Hybridization and Luminex detection (can refer to the conventional Luminex liquid chip for hybridization detection):
(1) preparation of microspheres working solution: take each kind of microspheres coupled with the probes specific for kRas codon 12 in the embodiment 1, wherein the probes are nucleotide sequences SEQ ID NO 1-8 (probe names: 60C12W1, 60C12WE1, 60C12MEE1 , 60C12ME2, 60C12ME3, 60C12ME4, 60C12ME5, 60C12ME6), these microspheres are evenly mixed by vortex for 30seconds and sonication for 30seconds, then the desired microspheres are added into the sterilized microfuge tube (0.5ul per reaction, and 2000 microspheres per ul for each kind of microsphere), then add 1.5X hybridization buffer (32.5ul per reaction) and TE buffer (14ul per reaction), and this is the microspheres working solution;
(2) according to the sample condition, the marker pen is used to mark the 96 well plate (hybridization plate) (negative control wells marked as "N"), and then add 47ul of microspheres working solution into each well;
(3) add 3ul of corresponding hybridization sample to the sample wells (the second PCR products); add 3ul of TE to the "N" well;
(4) put the hybridization plate into the PCR instrument, proceed with denaturation for 3min at 95 °C; and then hybridization at 60 °C for 15min;
(5) prepare the SA-PE (streptavidin phycoerythrin) working solution (10ug/ml) by using 1X hybridization buffer solution, and then the SA-PE working solution is preheated at 60 °C in water bath;
(6) add the SA-PE working solution (10ug/ml) which has been already preheated to the hybridization plate, 25ul for each well;
(7) put the hybridization plate into the PCR instrument for reaction for 5min at 60 °C;
(8) set the temperature for Luminex instrument tray to 60 °C, set instrument parameters according to Luminex instrument (read 100 microspheres for each sample, the reading time is 25sec). The hybridization plate is placed in the tray of Luminex instrument, and the sample MFI values are detected.

### 3. Result Analysis

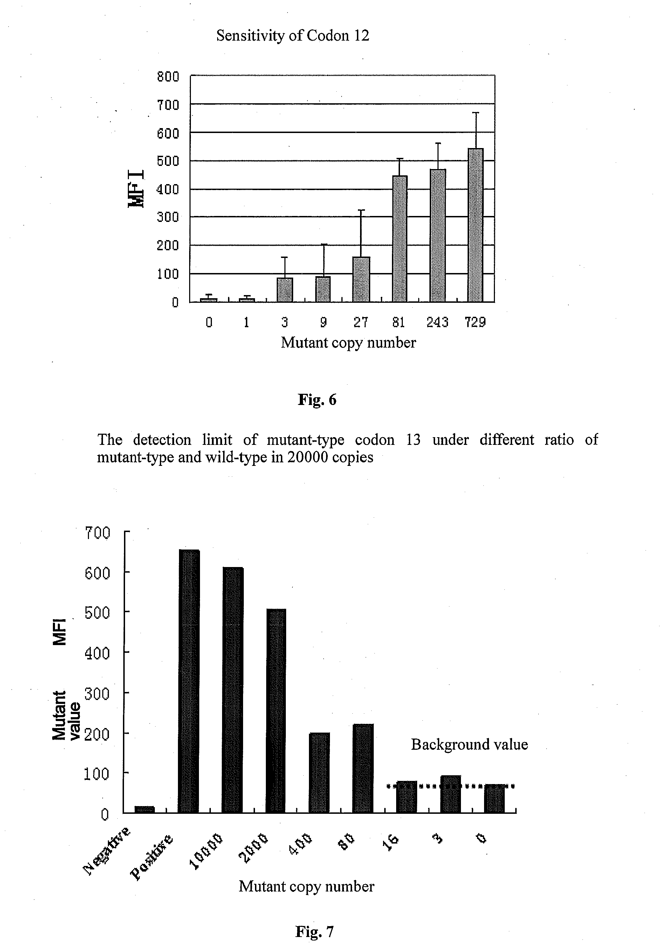

Figure 6 shows the results of hybridization detection, MFI value beyond 100 is the valid value. For 3 and 9 copies, it was only detected once among 4 detections, the average value of MFI is below 100 (SD value is relatively high). For 81 copies, it was detected among all of the 4 detections, and with good reproducibility (SD value is lower). These results demonstrated that the detection for low copy number has poor reproducibility, it has good reproducibility for detection of copies which beyond 80.

### Embodiment 4, experiment for detecting the mutant-type in wild-type kRas codon 12

In order to detect small amount of mutant-type in a large amount of wild-type, different ratios of kRas codon 12 wild-type and mutant-type are mixed to be the template (total copy number is 20000), which are proceeded with restriction digestion and enrichment respectively, then with the Luminex detection, and duplicate wells is made for each ratio. Two rounds of PCR amplification, restriction enzyme enrichment, microsphere coupling, hybridization and luminex detection methods are the same as embodiment 3.

The detected results are shown as Fig. 7 (MFI value beyond 100 is the valid value):
The detection results indicate that: 80 copies of plasmid containing mutant-type codon 12 can be detected in 20000 copies of wild-type codon 12. The results of this experiment show that the method of introducing restriction site by PCR can be used to detect small amount of mutant-type kRas gene from a large amount of wild-type kRas gene.

### Embodiment 5

### The detection liquid chip of kRas gene mutation is used to detect the colorectal cancer

### 1. Preparation of samples to be detected (the nucleic acid of the plasma, the serum, and the supernatant of the pleural fluid can be extracted from the following method):

Referring to the specification ofAxyPrep whole blood genomic DNA mini preparation kit, the detailed steps are as follows:
(1) get an amount of 2.5ml of anticoagulant venous blood or pleural effusion, and add 300µl of supernatant liquid into a 1.5ml clean and aseptic microfuge tube;
(2) add 500µl of AP1 buffer into the microfuge tube, and mix evenly by vortex vibration;
(3) add 100µl of AP2 buffer, and mix evenly by vortex vibration;
(4) perform centrifugation at 12, 000rpm for 10 minutes at room temperature;
(5) transfer the supernatant carefully to the absorption column AxyPrep on the 2ml collection tube, close the cover, and perform centrifugation at 6, 000rpm for 1 minute;
(6) drain the waste liquid in the waste-liquid collection tube, and wash once by using 800µl of W1 buffer, and perform centrifugation at 6, 000rpm for 1 minute;
(7) drain the waste liquid in the waste-liquid collection tube, add 800µl of W2 buffer, and perform centrifugation at 12, 000rpm for 1 minute; drain the waste liquid in the waste-liquid collection tube, and add 500µl of W2 buffer, and perform centrifugation at 12, 000rpm for 1 minute, and discard the waste liquid;
(8) put the absorption column AxyPrep back to the collection tube, and perform centrifugation at 12, 000rpm for 1 minute;
(9) put the absorption column AxyPrep into a 1.5ml clean and aseptic microfuge tube, and add 40µl of TE buffer, and dispose at the room temperature for 1 minute, and perform centrifugation at 12, 000rpm for 1 minute to elute DNA, perform electrophoresis detection, and preserved at -20°C.

### II. Enrichment by restriction digestion and PCR amplification of the samples to be detected

The enrichment for mutant-type of codon 12 by restriction digestion and PCR amplification is the same as the Embodiment 3.

The enrichment for mutant-type of codon 13 by restriction digestion and PCR amplification is as follows:

### 1. The first PCR amplification

### PCR reaction system

| Condon 13 reaction system | Per reaction (ul) |
|---|---|
| Sterile ddH2O | 28.8 |
| 5xColorless GoTaq Flexi Buffer | 10 |
| dNTP Mixture (each 2.5mM) | 2 |
| MgCl₂ (25mM) | 5 |
| Codon 13 primer F : K13F1 (10uM) | 1 |
| Codon 13 primer R : K13R (10uM) | 1 |
| GoTaq Hot Start polymerase ( 5U/ul) | 0.2 |
| DNA Template | 2 |
| Total volume | 50 |

### PCR reaction conditions:

| Steps | Temperature | Time | Cycle number |
|---|---|---|---|
| Initial denaturation | 94 °C | 3 min | 1 |
| Denaturation | 94 °C | 20 sec | 30 |
| Anneal | 60 °C | 30 sec | |
| Extension | 72 °C | 20 sec | |
| Extension | 72 °C | 10 min | 1 |
| Preservation | 4°C | ∞ | 1 |

### 2. NarI restriction digestion of PCR products:

### Reaction system is as follows:

| Sample system (Incubated at 37°C for 2 hours) | Per reaction (ul) |
|---|---|
| 10×Buffer G | 5 |
| BSA | 0.5 |
| NarI endonuclease (10U/ul) | 0.5 |
| PCR products | 10 |
| Sterile ddH2O | 34 |
| Total volume | 50 |

### 3. The second PCR amplification

### PCR reaction system

| Codon 12 reaction system | Per reaction (ul) |
|---|---|
| Sterile ddH2O | 28.8 |
| 5xColorless GoTaq Flexi Buffer | 10 |
| dNTP Mixture (each 2.5mM) | 2 |
| MgCl₂ (25mM) | 5 |
| Codon 13 primer F : K13F2 (10uM) | 1 |
| Codon 13 primer R : K13R-bio (10uM) | 1 |
| GoTaq Hot Start polymerase (5U/ul) | 0.2 |
| DNA Template | 2 |
| Total Volume | 50 |

### PCR reaction condition:

| Step | Temperature | Time | Cycle number |
|---|---|---|---|
| Initial denaturation | 94 °C | 3 min | 1 |
| Denaturation | 94 °C | 20 sec | 30 |
| Anneal | 60°C | 30 sec | |
| Extension | 72 °C | 20 sec | |
| Final extension | 72 °C | 10 min | 1 |
| Preservation | 4°C | ∞ | 1 |

The enrichment for mutant-type of codon 61 by BclI restriction digestion and PCR amplification is as follows:

### 1. The first PCR amplification (introducing restriction sites of BcII)

### PCR reaction system:

| Codon 61 reaction system | Per reaction (ul) |
|---|---|
| Sterile ddH₂O | 28.8 |
| 5xColorless GoTaq Flexi Buffer | 10 |
| dNTP Mixture (each 2.5mM) | 2 |
| MgCl₂ (25mM) | 5 |
| Codon 61 primer F : K61BcF (10uM) | 1 |
| Codon 61 primer R : K61BcR1 (10uM) | 1 |
| GoTaq Hot Start polymerase (5U/ul) | 0.2 |
| DNA Template | 2 |
| Total Volume | 50 |

### PCR reaction condition:

| Step | Temperature | Time | Cycle number |
|---|---|---|---|
| Initial denaturation | 94 °C | 3 min | 1 |
| Denaturation | 94 °C | 20 sec | 25 |
| Anneal | 60 °C | 30 sec | |
| Extension | 72 °C | 20 sec | |
| Extension | 72 °C | 10 min | 1 |
| Preservation | 4°C | ∞ | 1 |

### 2. BclI restriction digestion of PCR products

### Reaction system is as follows:

| Sample system (Incubated at 50°C for 2 hours) | Per reaction (ul) |
|---|---|
| 10×Buffer G | 5 |
| BSA | 0.5 |
| BelI endonuclease (10U/ul) | 0.5 |
| PCR products | 10 |
| Sterile ddH₂O | 34 |
| Total Volume | 50 |

### 3. The second PCR amplification (introducing restriction sites of BclI)

### PCR reaction system:

| **Codon 61 reaction system** | **Per reaction (ul)** |
|---|---|
| Sterile ddH₂O | 28.8 |
| 5×colorless GoTaq Flexi Buffer | 10 |
| dNTP Mixture (each 2.5mM) | 2 |
| MgCl₂ (25mM) | 5 |
| Codon 61primer F : K61BcF(10uM) | 1 |
| Codon 61 primer R : K61BcR1-b (10uM) | 1 |
| GoTaq Hot Start polymerase (5U/ul) | 0.2 |
| DNA Template | 2 |
| Total Volume | 50 |

### PCR reaction condition:

| Steps | Temperature | Time | Cycle number |
|---|---|---|---|
| Initial denaturation | 94 °C | 3 min | 1 |
| Denaturation | 94 °C | 20 sec | 30 |
| Anneal | 60°C | 30 sec | |
| Extension | 72 °C | 20 sec | |
| Final extension | 72 °C | 10 min | 1 |
| Preservation | 4°C | ∞ | 1 |

The enrichment for mutant-type codon 61 by BglII restriction digestion and PCR amplificationis as follows:

### 1. The first PCR amplification (introducing restriction sites of BglII)

### PCR reaction system:

| Codon 61 reaction system | Per reaction (ul) |
|---|---|
| Sterile ddH₂O | 28.8 |
| 5 xColorless GoTaq Flexi Buffer | 10 |
| dNTP Mixture (each 2.5mM) | 2 |
| MgCl₂ (25mM) | 5 |
| Codon 61 primer F : K61BgF (10uM) | 1 |
| Codon 61 primer R : K61BgR1 (10uM) | 1 |
| GoTaq Hot Start polymerase (5U/ul) | 0.2 |
| DNA Template | 2 |
| Preservation | 50 |

### PCR reaction condition:

| Steps | Temperature | Time | Cycle number |
|---|---|---|---|
| Initial denaturation | 94 °C | 3 min | 1 |
| Denaturation | 94 °C | 20 sec | 25 |
| Anneal | 60 °C | 30 sec | |
| Extension | 72 °C | 20 sec | |
| Extension | 72 °C | 10 min | 1 |
| Preservation | 4°C | ∞ | 1 |

### 2. BglII restriction digestion of PCR products

The reaction system is as follows:

| Sample system (Incubated for 2 hours at 37°C) | Per reaction (ul) |
|---|---|
| 10xBuffer G | 5 |
| BSA | 0.5 |
| BglII endonuclease (10U/ul) | 0.5 |
| PCR products | 10 |
| Sterile ddH₂O | 34 |
| Total Volume | 50 |

### 3. The second PCR amplification (introducing restriction sites of BglII)

### PCR reaction system:

| Codon 61 reaction system | Per reaction (ul) |
|---|---|
| Sterile ddH₂O | 28.8 |
| 5xColorless GoTaq Flexi Buffer | 10 |
| dNTP Mixture (each 2.5mM) | 2 |
| MgCl₂ (25mM) | 5 |
| Codon 61 primer F : K61BgF2(10uM) | 1 |
| Codon 61primer R : K61BgR1-b (10uM) | 1 |
| GoTaq Hot Start polymerase (5U/ul) | 0.2 |
| DNA Template | 2 |
| Total Volume | 50 |

### PCR reaction condition:

| Steps | Temperature | Time | Cycle number |
|---|---|---|---|
| Initial denaturation | 94 °C | 3 min | 1 |
| Denaturation | 94 °C | 20 sec | 30 |
| Anneal | 60°C | 30 sec | |
| Extension | 72 °C | 20 sec | |
| Final extension | 72 °C | 10 min | 1 |
| Preservation | 4°C | ∞ | 1 |

### II. Luminex detection

Hybridization and Luminex detection
(1) preparation of microsphere working solution: each kind of the microspheres coupled with the probes specific for kRas codon 12, codon 13, and codon 61 in the Embodiment 1 (the probes are nucleotide sequence SEQ ID NO. 1-18) is mixed evenly by vortex for 30 seconds and sonication for 30 seconds, and then take the microspheres desired into the sterile microfuge tube (0.5ul per reaction, and 2000 microspheres/ul for each kind of microsphere), and add 1.5X hybridization buffer (32.5ul per reaction) and TE buffer (14ul per reaction), and this is the microsphere working solution.
(2) The other hybridization and Luminex detection steps are the same as that of the hybridization and Luminex detection in the Embodiment 3.

### III. Detection results and data analysis

The reaction products were detected by the Luminex analyzer. Its detection results are indicated in Table 1. The cut-off value of mutant-type of codon 12 is Mean Fluorescence Intensity (MFI) value 100. While the MFI value of mutant-type probes of codon 12 is beyond 100, it is judged that the sample has mutants-type of codon 12, or else the sample is judged as the wild-type of codon 12. The cut-off value of mutant-type of codon 13 and codon 61 are MFI value 200. While the MFI value of mutant-type probes of codon 13or codon 61 is beyond 200, it is judged that the existence of mutations of codon 13 or codon 61 in the sample, or else the sample is judged as the wild-type of codon 13 or codon 61.

According to the above criteria, among 10 samples detected in this embodiment, 6 samples have codon 12 mutations (sample No. 2, 3, 4, 5, 6 and 9), 4 samples have codon 13 mutations (sample No. 1, 4, 5 and 10), 3 samples have codon 61 mutations (sample No. 1, 2 and 3).

**Table 1, the detection results of serum samples**

| NO. | positive | negative | 12W | 12M | 13W | 13M | 61W | 61M |
|---|---|---|---|---|---|---|---|---|
| | control | control | ( MFI ) | ( MFI ) | (MFI) | ( MFI ) | ( MFI ) | ( MFI ) |
| | (MFI) | (MFI) | | | | | | |
| 1 | 3395 | 26 | 1751 | 70 | 469 | 989 | 369 | 789 |
| 2 | 3545 | 30 | 362 | 993 | 1256 | 116 | 401 | 921 |
| 3 | 4053 | 15 | 473 | 839 | 1941 | 95 | 451 | 1036 |
| 4 | 4456 | 17 | 332 | 903 | 569 | 1002 | 901 | 66 |
| 5 | 3986 | 24 | 436 | 876 | 454 | 1153 | 1061 | 81 |
| 6 | 3937 | 20 | 256 | 754 | 1008 | 71 | 1161 | 90 |
| 7 | 4369 | 11 | 1066 | 65 | 961 | 99 | 986 | 95 |
| 8 | 4255 | 24 | 983 | 43 | 1171 | 128 | 943 | 86 |
| 9 | 3061 | 15 | 346 | 818 | 1225 | 86 | 861 | 79 |
| 10 | 4126 | 16 | 1169 | 48 | 623 | 954 | 1261 | 89 |

## Claims

1. Probes for detecting kRas gene mutations, comprising:
specific for kRas codon 12: a wild-type probe of SEQ ID NO.1, a wild-type probe of SEQ ID NO.2 introducing restriction site BstOI, and a mutant-type probe of one or more nucleotide sequences selected from SEQ ID NO. 3- SEQ ID NO.8 introducing restriction site BstOI; and/ or
specific for kRas codon 13: a wild-type probe of SEQ ID NO. 9, a wild-type probe of SEQ ID NO.10 introducing restriction site NarI, and a mutant-type probe of one or both nucleotide sequences selected from SEQ ID NO.11 and SEQ ID NO.12 introducing restriction site NarI; and/ or;
specific for kRas codon 61: a wild-type probe of SEQ ID NO.13, a wild-type probe of SEQ ID NO.14 introducing restriction site BclI, a wild-type probe of SEQ ID NO.15 introducing restriction site BglII, and a mutant-type probe of one or more nucleotide sequences selected from SEQ ID NO.16- introducing restriction site BclI, and SEQ ID NO.18 introducing restriction site BglII.

2. A liquid chip for detecting kRas gene mutations, comprising:
A. microspheres coupled with probes:
microspheres respectively coupled with amino-substituted wild-type probes specific for kRas codon 12, wherein the wild-type probes are SEQ ID NO. 1 and SEQ ID NO. 2; and microspheres respectively coupled with amino-substituted mutant-type probes specific for kRas codon 12, wherein the mutant-type probes are one or more nucleotide sequences selected from SEQ ID NO. 3- SEQ ID NO.8; and/or
microspheres respectively coupled with amino-substituted wild-type probes specific for kRas codon 13, wherein the wild-type probes are SEQ ID NO.9 and SEQ ID NO.10; and microspheres respectively coupled with amino-substituted mutant-type probes specific for kRas codon 13, wherein the mutant-type probes are one or both nucleotide sequences selected from SEQ ID NO.11 and SEQ ID NO.12; and/or
microspheres respectively coupled with amino-substituted wild-type probes specific for kRas codon 61, wherein the wild-type probes are SEQ ID NO.13 SEQ ID NO. 14 and SEQ ID NO.15; and microspheres respectively coupled with amino-substituted mutant-type probes specific for kRas codon 61, wherein the mutant-type probes are one or more nucleotide sequences selected from SEQ ID NO.16 SEQ ID NO.17 introducing restriction site BelI, and SEQ ID NO.18 introducing restriction site BglII;
wherein a spacer is connected between the nucleotide sequence of each kind of the above-mentioned probes and the amino group, and microspheres coupled with different probes have different color codes; and
B. primers: primers for amplifying target sequences enriched for mutant alleles of kRas codons 12, 13 and/or 61 to be detected, said target sequence being biotin-labeled at the terminal.

3. The liquid chip for detecting kRas gene mutations according to claim 2, wherein the primers comprise:
primers for amplifying the target sequences enriched for mutant alleles of kRas codons 12, wherein the primers are SEQ ID NO. 19 - SEQ ID NO. 21, and at least one of the primers is biotin-labeled at the terminal; and/or
primers for amplifying the target sequences enriched for mutant alleles of kRas codons 13, wherein the primers are SEQ ID NO. 22 - SEQ ID NO. 25, and at least one of the primers is biotin-labeled at the terminal; and/or
primers for amplifying the target sequences enriched for mutant alleles of kRas codons 61, wherein the primers are SEQ ID NO. 26 - SEQ ID NO. 32, and at least one of the primers is biotin-labeled at the terminal.

4. The liquid chip for detecting kRas gene mutations according to claim 2, wherein the spacer is an oligonucleotide consisting of 5 to 40 deoxythymidylates.

5. A method for detecting kRas gene mutations, using the liquid chip for detecting kRas gene mutations according to claim 2, comprising the following steps:
(1) performing a first PCR amplification for wild-type and mutant-type DNA samples by using PCR primers with resctriction site;
(2) performing restriction digestion to products obtained from the first PCR amplification of the step (1);
(3) performing a second PCR amplification for the mutant-type kRas gene by using products obtained after the restriction digestion as template;
(4) hybridizing products obtained from the second PCR amplification to the corresponding probes coupled to microspheres of claim 2;
(5) performing reaction by adding streptavidin-phycoerythrin after the hybridization reaction of the step (4), and then performing signal detection.

6. The method for detecting kRas gene mutations according to claim 5, wherein the primers used in the first PCR amplification are:
a pair of primers of SEQ ID NO. 19 and SEQ ID NO.20 specific for mutant alleles of kRas codon 12; and/or
a pair of primers of SEQ ID NO.22 and SEQ ID NO.24 specific for mutant alleles of kRas codon 13; and/or
a pair of primers of SEQ ID NO.26 and SEQ ID NO.27 specific for mutant alleles of kRas codon 61, and a pair of primers of SEQ ID NO.29 and SEQ ID NO.31 specific for mutant alleles of kRas codon 61.

7. The method for detecting kRas gene mutations according to claim 5, wherein the primers used in the second PCR amplification are:
a pair of primers of SEQ ID NO.19 and SEQ ID NO.21 specific for mutant alleles of kRas codon 12; and/or
a pair of primers of SEQ ID NO.23 and SEQ ID NO.25 specific for mutant alleles of kRas codon 13; and/or
a pair of primers of SEQ ID NO.26 and SEQ ID NO.28 specific for mutant alleles of kRas codon 61, and a pair of primers of SEQ ID NO.30 and SEQ ID NO.32 specific for mutant alleles of kRas codon 61, wherein one of each pair of the above-mentioned primers is biotin-labeled at the 5'terminal.
